# EUROPÄISCHE PATENTANMELDUNG

(11) **EP 4 635 394 A1**
(43) Veröffentlichungstag der Anmeldung: **22.10.2025**
(21) Anmeldenummer: 25170807.9
(22) Anmeldetag: 15.04.2025
(51) Int. Cl.: A61B 1/00, A61B 1/04, G02B 7/00, G02B 26/00

(54) **FILTERWECHSELVORRICHTUNG FÜR EINE ENDOSKOPISCHE KAMERA, KAMERAKOPF FÜR EIN ENDOSKOP UND NACHRÜSTSATZ ZUM NACHRÜSTEN EINES KAMERAKOPFES UND/ODER EINES ENDOSKOPS**

(30) Priorität: 18.04.2024 DE 102024110878
(71) Anmelder: Karl Storz SE & Co. KG, 78532 Tuttlingen (DE)
(72) Erfinder: LEMPERLE, Max, 78532 Tuttlingen (DE); HUBER, Florian, 78532 Tuttlingen (DE); DAVID, Jonas, 78532 Tuttlingen (DE)

(57) **Zusammenfassung**

Die Erfindung betrifft eine Filterwechselvorrichtung für eine endoskopische Kamera, wobei die Filterwechselvorrichtung ein Gehäuse mit mindestens einem ersten Gehäuseteil, einen optischen Durchgang entlang einer optischen Achse, ein drehbares Antriebszahnrad und mindestens zwei Filterhalterarme mit jeweils einer Filteraufnahme für einen optischen Filter mit einer Filtermittelachse aufweist, wobei die mindestens zwei Filterhalterarme jeweils mittels einer Rotationswelle mit einer Rotationsachse drehbar an dem ersten Gehäuseteil angeordnet sind, wobei die jeweilige Rotationsachse beabstandet zu der Filtermittelachse des jeweiligen Filterhalterarms ist und die Rotationsachsen parallel zueinander angeordnet sind, wobei an jedem Filterhalterarm ein Filterzahnrad um die jeweilige Rotationswelle angeordnet ist und das drehbare Antriebszahnrad in das jeweilige Filterzahnrad eingreifbar ist, sodass bei einem alleinigen Antreiben des drehbaren Antriebszahnrades mittels Eingreifen in die Filterzahnräder die mindestens zwei Filterhalterarme gleichzeitig drehbar und/oder in den optischen Durchgang einschwenkbar und/oder ausschwenkbar sind. Des Weiteren betrifft die Erfindung einen Kamerakopf und einen Nachrüstsatz zum Nachrüsten eines Kamerakopfs und/oder eines Endoskopes.

## Beschreibung

Die Erfindung betrifft eine Filterwechselvorrichtung für eine endoskopische Kamera, wobei die Filterwechselvorrichtung eine Gehäuse mit mindestens einem ersten Gehäuseteil, einen optischen Durchgang entlang einer optischen Achse, ein drehbares Antriebszahnrad und mindestens zwei Filterhalterarme mit jeweils einer Filteraufnahme für einen optischen Filter mit einer Filtermittelachse aufweist, wobei die mindestens zwei Filterhalterarme jeweils mittels einer Rotationswelle mit einer Rotationsachse drehbar an dem ersten Gehäuseteil angeordnet sind, wobei die jeweilige Rotationsachse beabstandet zu der Filtermittelachse des jeweiligen Filterhalterarms ist und die Rotationsachsen parallel zueinander angeordnet sind. Des Weiteren betrifft die Erfindung einen Kamerakopf für ein Endoskop und einen Nachrüstsatz zum Nachrüsten eines Kamerakopfes und/oder eines Endoskopes.

In medizinischen und nicht-medizinischen Anwendungen werden Beobachtungsinstrumente wie Endoskope eingesetzt, um innere Hohlräume eines menschlichen oder tierischen Körpers oder eines industriellen, technischen Objektes, wie eine Rohrleitung, zu untersuchen. Für die Bildgebung kann ein Kamerakopf aufweisend einen Bildsensor zusammen mit dem Endoskop eingesetzt werden. Um die Bildqualität zu verbessern und/oder verschiedene Beobachtungsmodi zu ermöglichen, ist es bekannt, verschiedene Filter in den Strahlengang des Beobachtungsinstrumentes einzubringen.

Zum Beispiel wird in der Fluoreszenz-Bildgebung das zu untersuchende Objekt einem Licht mit einer Anregungsstrahlung ausgesetzt, welche ein vorher auf das Objekt aufgebrachtes oder bereits vorhandenes Fluorophor anregt, Licht einer bestimmten Emissionswellenlänge auszusenden, wobei die Anregungswellenlänge und die Emissionswellenlänge sich üblicherweise unterscheiden. Normalerweise ist die Emissionswellenlänge länger als die Anregungswellenlänge. Das abgegebene Emissionslicht ist üblicherweise deutlich schwächer als andere Lichtquellen, wie das Anregungsfluoreszenzlicht oder das bildgebende Weißlicht. Aus diesen Gründen ist es notwendig, unerwünschte Wellenlängenbänder mittels eines Filters herauszufiltern, sodass möglichst nur das gewünschte Spektrum und/oder die Emissionswellenlänge des Fluorophors den Kamerakopf im Fluoreszenzmodus erreicht. Zum Wechsel zwischen verschiedenen Beobachtungsmodi werden üblicherweise zwei oder mehrere Filter nacheinander in den Strahlengang der Beobachtungsoptik eingebracht, wozu prinzipiell verschiedene Filterwechsler bekannt sind.

DE 101 57 057 A1 offenbart eine Vorrichtung zum Positionieren zumindest eines optischen Bauelementes innerhalb eines endoskopischen Systems mit einem Gehäuse, durch welches die optische Achse des endoskopischen Systems verläuft und in dem das zumindest eine Bauelement angeordnet ist, welches um eine im Wesentlichen parallel zu einer Längsachse des Gehäuses verlaufenden Schwenkachse in den Strahlengang einschwenkbar und aus diesem wieder ausschwenkbar ist, wobei das zumindest eine Bauelement, beispielsweise ein Filter für einen speziellen spektralen Wellenlängenbereich, an einem um die Schwenkachse schwenkbaren Träger angeordnet ist. Hierbei ist ein kleinster Abstand einer Innenwand des Gehäuses von der Schwenkachse kleiner als ein größter Abstand der Schwenkachse zu einer Außenkante des zumindest einen Bauelementes. Durch die nähere Anordnung der Schwenkachse jedes schwenkbaren Trägers zu der Innenwand des Gehäuses ist die Anzahl der Träger zum Halten möglichst unterschiedlicher Filter durch die räumlich weitreichende Schwenkbewegung innerhalb des limitierten Raumes des Gehäuses begrenzt. Zudem ist das Gehäuse fest mit einem Gehäuse des optischen Kopfes des Endoskopes verbunden. Weiterhin nachteilig ist, dass durch die räumlich nähere Anordnung der Schwenkachse an der Innenwand des Gehäuses eine Vielzahl von beweglichen Einzelteilen erforderlich ist, um mehrere Filter in den Strahlengang einzubringen. Dies hat einen erhöhten Kosten- und Montageaufwand zur Folge. Zudem erhöht sich dadurch das Risiko von Verschleiß, Ungenauigkeiten in der jeweiligen Schwenkbewegung und folglich von Fehlfunktionen.

Aus dem anmeldereigenen Stand der Technik (deutsches Anmeldeaktenzeichen 10 2022 131 502.9) ist eine Filterwechselvorrichtung für einen endoskopischen Kamerakopf mit mindestens drei optischen Filtern bekannt, welche eine Nut mit einer nicht kreisförmigen Führungsbahn und einem Drehelement aufweist, wobei durch Drehen des Drehelementes nacheinander in der Nut angeordnete Träger für jeweils ein Filter bewegt oder eine Schwenkbewegung eines Trägerarms mittels eines partiell in der Nut angeordneten Führungselementes aufgrund einer zumindest partiellen Bewegung entlang der nicht kreisförmigen Führungsbahn in und aus dem Strahlengang erfolgt. Nachteilig hierbei ist, dass zur Anordnung von mehreren Filtern jeweils ein Trägerarm mit einem Führungselement notwendig ist, sodass es aufgrund der größeren Anzahl von Führungselementen oder direkt in der Nut angeordneten Filterträgern durch die auftretenden Reibungskräfte zu störenden Einflüssen auf die Funktion der Filterwechselvorrichtung kommen kann. Hierbei können die hohen Reibungskräfte ein Verklemmen des Bewegungsmechanismus und/oder einen Verschleiß mit negativer Beeinflussung auf das Ein- und Ausschwenkverhalten und eine Rastposition bewirken, sodass eine Fehlfunktion oder ein Defekt der Filterwechselvorrichtung auftreten kann. Zudem überlappen sich die schwenkbaren Trägerarme, sodass entlang der optischen Achse ein größerer Bauraum notwendig ist.

Allgemein nachteilig bei bekannten Filterwechselvorrichtungen mit schwenkbaren Trägerarmen ist, dass entweder nur ein Trägerarm in den optischen Eingang ein- und ausgeschwenkt wird und die anderen Trägerarme sich in einer Ruheposition befinden oder gleichzeitig zwei oder mehrere schwenkbare Trägerarme mit gegenläufigen Bewegungen des Ein- und Ausschwenkens koordiniert werden müssen. Dies führt entweder zu einem Zeitverlust beim Filterwechsel durch die Bewegung nur eines Trägerarms oder vor allem bei mehr als drei schwenkbaren Trägerarmen zu einem hohen Aufwand aufgrund der einzelnen, jedoch zueinander abgestimmten gleichzeitigen Bewegungen und/oder Antriebe der Trägerarme. Bei einer gleichzeitigen Bewegung von zwei oder mehreren Trägerarmen ist üblicherweise ein großer Bauraum quer zur optischen Achse und somit ein großer Durchmesser der Filterwechselvorrichtung notwendig, um eine Kollision der sich gleichzeitig bewegenden Trägerarme zu vermeiden.

Aufgabe der Erfindung ist es, den Stand der Technik zu verbessern.

Gelöst wird die Aufgabe durch eine Filterwechselvorrichtung für eine endoskopische Kamera, wobei die Filterwechselvorrichtung ein Gehäuse mit mindestens einem ersten Gehäuseteil, einen optischen Durchgang entlang einer optischen Achse, ein drehbares Antriebszahnrad und mindestens zwei Filterhalterarme mit jeweils einer Filteraufnahme für einen optischen Filter mit einer Filtermittelachse aufweist, wobei die mindestens zwei Filterhalterarme jeweils mit einer Rotationswelle mit einer Rotationsachse drehbar an dem ersten Gehäuseteil angeordnet sind, wobei die jeweilige Rotationsachse beabstandet zu der Filtermittelachse des jeweiligen Filterhalterarms ist und die Rotationsachsen parallel zueinander angeordnet sind, wobei an jedem Filterhalterarm ein Filterzahnrad um die jeweilige Rotationswelle angeordnet ist und das drehbare Antriebsrad in das jeweilige Filterzahnrad eingreifbar ist, sodass bei einem alleinigen Antreiben des drehbaren Antriebszahnrades mittels Eingreifen in die Filterzahnräder die mindestens zwei Filterhalterarme gleichzeitig drehbar und/oder in den optischen Durchgang einschwenkbar und/oder ausschwenkbar sind.

Somit wird eine Filterwechselvorrichtung bereitgestellt, mit welcher mindestens zwei oder mehrere Filter aufgrund der gleichzeitigen Bewegung aller Filterhalterarme schnell und direkt aufeinander folgend in den Strahlengang einer endoskopischen Kamera einschwenkbar sind. Da das drehbare Antriebszahnrad als einziger Antrieb zeitgleich in alle Filterzahnräder eingreift und somit die Filterhalterarme gleichzeitig bewegt, finden simultan schnelle, präzise und effiziente Ein- und Ausschwenkbewegungen der Filterhalterarme statt. Folglich ist ein schneller und aufgrund einer vorgegebenen Reihenfolge des Eingreifens des einzigen Antriebszahnrades in die Filterzahnräder ein eindeutiger Wechsel zwischen verschiedenen Filtern ermöglicht. Vor allem sind durch das einfache Drehen des einzigen Antriebsrades im Uhrzeigersinn und/oder gegen den Uhrzeigersinn eine vorgegebene Abfolge der Filter und ein schneller Wechsel zwischen verschiedenen Filtern und somit schnell nacheinander verschiedene Beobachtungsmodi und Bildgebungen ermöglicht.

Es ist besonders vorteilhaft, dass die Filterwechselvorrichtung eine gleichzeitige Bewegung aller Filterhalterarme ermöglicht, dazu jedoch nur ein einziges Antriebszahnrad und die gewünschte Anzahl an Filterhalterarme mit jeweils einem Filterzahnrad benötigt, ohne dass mehrere aufwändige, zusätzliche Übertragungsbauteile notwendig sind. Zudem wird dadurch der Verschleiß auch bei langen Betriebszeiten minimiert und somit eine lange Standzeit und eine stabile Funktion der Filterwechselvorrichtung gewährleistet.

Aufgrund des einfachen Antriebsmechanismus und der Vorgabe des Bewegungsraums durch das eine drehbare Antriebszahnrad und die Dimensionen der Filterhalterarme mit den jeweiligen Filterzahnrädern, ist die Filterwechselvorrichtung einfach und zuverlässig automatisierbar und in ihrer Größe und/oder an die Anzahl der Filterhalterarme anpassbar.

Durch eine Anordnung der Filterzahnräder und des einzigen Antriebszahnrades in der Art eines Umlaufrädergetriebes bewegen sich alle Filterzahnräder kontinuierlich und nacheinander entlang der Zähne und/oder des Außenumfangs des Antriebszahnrades, wobei jeder Filterhalterarm und somit das zugehörige optische Filter eine vollständige Kreisbewegung zum Ein- und Ausschwenken durchführt. Da mittels des einzigen Antriebszahnrades die Bewegungsrichtung und der Bewegungsablauf der Filterhalterarme über das Eingreifen in die Filterzahnräder vorgegeben ist, sind durch eine räumliche Anordnung der Rotationswellen und/oder -achsen der Filterhalterarme in Relation zum Antriebszahnrad die simultanen Bewegungen der Filterhalterarme optimal einstellbar und/oder koordinierbar und somit eine Kollision der Filterhalterarme vermeidbar. Es ist besonders vorteilhaft, dass die Filterhalterarme und somit die Filter gerade aufeinander abgestimmte und aufeinander folgende Kreisbewegungen durchführen und sich somit nicht aneinander vorbei bewegen und/oder in ihrem jeweiligen Schwenkradius überlappende Schwenkbewegungen mit dem Nachteil eines hohen Kollisionsrisikos durchführen. Dagegen wird in der erfindungsgemäßen Filterwechselvorrichtung gerade eine Kollision vermieden, da die Filterhalterarme sich entlang der Kreisbahn des Antriebszahnrades in kontinuierlichen Kreisbewegungen nacheinander bewegen und da ihre Rotationsachsen stets parallel zueinander angeordnet sind.

Durch die aufeinander abgestimmten räumlichen Anordnungen und die gleichzeitigen Bewegungen der Filterhalterarme und durch deren Anordnung mittels der Rotationswelle nur an einem Gehäuseteil und somit nur innenliegend auf einer Seite des Gehäuses ist eine sehr kompakte Bauform der Filterwechselvorrichtung realisiert. Vor allem weist die Filterwechselvorrichtung dadurch eine geringe Abmessung entlang der optischen Achse und einen geringen Durchmesser quer zur optischen Achse auf.

Ein wesentlicher Gedanke der Erfindung beruht darauf, mindestens zwei um ihre jeweilige Rotationsachse rotierbare Filterhalterarme nur an einem distalseitigen oder proximalseitigen Gehäuseteil anzuordnen und über deren jeweilige Filterzahnräder gleichzeitig und koordiniert zueinander mittels eines einzigen Antriebszahnrades anzutreiben und zu bewegen, wobei aufgrund des kontinuierlichen Eingreifens der Zähne des Antriebszahnrades in die Zähne aller Filterzahnräder sich alle Filterhalterarme gleichzeitig und abgestimmt aufeinander bewegen und/oder ein- und/oder ausschwenken. In Zusammenwirken mit den Anordnungen der jeweils unendlich rotierbaren, mindestens zwei Filterhalterarme an dem einen Gehäuseteil mittels einer jeweiligen Rotationswelle und des Eingreifens der Zähne des Antriebszahnrads in die Zähne des jeweiligen Filterzahnrades eines Filterhalterarms wird eine Abfolge und ein zeitlicher Verlauf der Bewegungen aller Filterhalterarme und eine Reihenfolge des Wechselns der Filter beim Antreiben des Antriebszahnrades vorgegeben. Somit wird eine Filterwechselvorrichtung bereitgestellt, mit welcher verschiedene Filter, insbesondere mehrere Fluoreszenzfilter und/oder ein Weißlichtfilter, nacheinander in den Strahlengang einer endoskopischen Kamera schnell, präzise und effizient in den optischen Durchgang ein- und ausschwenkbar sind. Dadurch ist eine lange Standzeit der Filterwechselvorrichtung gegeben. Neben dem schnellen Filterwechsel ist durch das Einsetzen von gleichen und/oder verschiedenen Filtern in die Filteraufnahmen der Filterhalterarme auch die Häufigkeit der Verwendung eines bestimmten Filters vorgebbar. Beispielsweise wird üblicherweise ein Beobachtungsmodus mit normalen Weißlicht häufiger verwendet als Fluoreszenzlicht. Vorteilhaft ist somit ein Weißlichtfilter jeweils vor und/oder nach einem Fluoreszenzfilter in Filteraufnahmen von nacheinander folgenden Filterhalterarme anordenbar, sodass neben einer gewünschten häufigeren Nutzung von Weißlich durch den jeweiligen Wechsel auf ein Weißlichtfilter dem Anwender auch eindeutig optisch der Wechsel zwischen zwei gleichen oder verschiedenen aufeinanderfolgenden anderen Filtern, beispielsweise zwei Fluoreszenzfiltern, anzeigbar ist. Somit wird das Risiko vermindert, dass unbeabsichtigt in einem falschen Beobachtungsmodus gearbeitet wird.

Folgendes Begriffliche sei erläutert:
Eine "Filterwechselvorrichtung" (auch "Filterwechsler" genannt) ist insbesondere eine Vorrichtung, mit welcher zumindest ein Filter von zwei oder mehreren Filtern in und aus dem optischen Strahlengang bewegbar ist. Mittels der Filterwechselvorrichtung werden zwei oder mehrere Filter insbesondere einzeln und nacheinander in den optischen Durchgang eingeschwenkt und/oder wieder ausgeschwenkt, wobei insbesondere simultane Bewegungen aller Filterhalterarme erfolgen. Die Filterwechselvorrichtung ist insbesondere manuell oder automatisch aktivierbar zum Wechseln der Filter durch Antreiben und/oder Drehen des drehbaren Antriebszahnrades. Somit ist ein Filter automatisch oder manuell durch Antreiben und/oder Drehen des Antriebszahnrades in den optischen Durchgang ein- und ausschwenkbar. Hierdurch kann ein Wechsel zwischen mindestens zwei Filteraufnahmen und/oder Filtern zweier Filterhalterarme erfolgen, deren Filterzahnräder mittels Eingreifen des Antriebszahnrades kontinuierlich bewegt werden. Die Filterwechselvorrichtung weist insbesondere mindestens zwei optische Filter, bevorzugt mindestens drei oder vier und optional weitere optische Filter, auf. Optional kann die Filterwechselvorrichtung auch eine Filteraufnahme aufweisen, welche kein optisches Filter aufweist und somit einen freien Durchlass durch den optischen Strahlengang ermöglicht. Somit kann mittels der Filterwechselvorrichtung auch eine leere Filteraufnahme in den optischen Durchgang und in den Strahlengang eingebracht werden. Ebenso kann der freie Durchgang statt Weglassen eines optischen Filters auch durch ein nicht filtrierendes optisches Element, wie eine Glasscheibe, ermöglicht sein. Eine Glasscheibe als Fenster kann auch eine Anti-Reflexions-Beschichtung aufweisen. Die Filterwechselvorrichtung kann insbesondere in einer Kamera integriert sein oder als separate Vorrichtung, beispielsweise ausgebildet als Aufschnappfilter, mit der Kamera und/oder einem Endoskop verbindbar sein. Für einen automatischen Filterwechsel kann die Filterwechselvorrichtung ein Bedienelement, beispielsweise einen Schalter an ihrer Außenoberfläche, aufweisen. Alternativ oder ergänzend zu einer optischen Erkennung des Filterwechsels durch den Anwender kann die Filterwechselvorrichtung auch ein Anzeigeelement und/oder einen Sensor, beispielsweise einen Hall-Sensor, aufweisen.

Ein "optisches Filter" (vereinfacht auch als "Filter" bezeichnet) ist insbesondere ein optisches Element, welches die einfallende Strahlung und/oder einfallenden Strahlen basierend auf spezifischen Eigenschaften, wie beispielsweise einer Wellenlänge, einem Polarisationszustand, einem Einfallswinkel und/oder einer Einfallsrichtung, selektiert und somit durchlässt oder am Durchlassen hindert. Ebenso kann ein optisches Filter die Eigenschaften des durchtretenden Lichtes verändern, beispielsweise durch Umwandlung von kreisförmig polarisiertem Licht in linearpolarisiertem Licht. Insbesondere kann ein optisches Filter ein spezifisches spektrales Wellenlängenband blockieren. Ein optisches Filter kann beispielsweise ein Verlaufsfilter, ein Kantenfilter, ein Polarisationsfilter oder ein Interferenzfilter sein. Ein Interferenzfilter weist insbesondere eine Beschichtung auf, welche ein Blockieren oder Durchlassen von Licht eines bestimmten Spektralbereiches bewirkt. Das optische Filter ist insbesondere als Beobachtungsfilter und/oder Detektionsfilter, Fluoreszenzbeobachtungsfilter oder Anregungsfilter einsetzbar. Das optische Filter weist insbesondere Glas oder ein kristallines Material auf. Das optische Filter kann planar oder als Filterlinse ausgebildet sein. Prinzipiell kann anstelle des optischen Filters auch ein anderes optisches Element, wie beispielsweise eine Linse, eine Blende, ein Polarisator oder ein ähnliches optisches Element, in der Filterwechselvorrichtung und/oder der Filteraufnahme angeordnet sein.

Unter "Weißlichtfilter" wird insbesondere verstanden, dass eine entsprechende Aufnahme und/oder Position frei von einem optischen Element ist oder ein optisches Element in einer entsprechenden Aufnahme und/oder Position frei von einer Filterfunktion ist, sodass das Licht und/oder Weißlicht insbesondere unverändert durchgelassen wird. Weißlicht wird von einem Weißlichtfilter insbesondere ohne Änderung seiner Lichteigenschaften, insbesondere der Wellenlängen, durchgelassen. Bei einem Weißlichtfilter kann es sich auch um ein Filter handeln, welches Nahinfrarotes Licht blockt. Ein "Weißlichtfilter" kann auch ein Filter sein, welcher das Licht filtriert, um eine Qualität des Bildes bei Beleuchtung mit weißem Licht zu verbessern. Hierfür kann z.B. ein BG39 Filter von der Firma Schott verwendet werden. Somit kann mittels eines Weißlichtfilters auch das durch einen Bildsensor und/oder eine Kamera erfasste Licht an eine Empfindlichkeitskurve und/oder eine spezifische Empfindlichkeit des menschlichen Auges angepasst werden.

Ein "Fluoreszenzbeobachtungsfilter" (auch als "Fluoreszenzfilter" bezeichnet) ist insbesondere ein optisches polychroitisches Interferenzfilter zum Trennen des emittierten Fluoreszenzlichtes von dem eingesetzten Anregungslicht. Somit blockiert das Fluoreszenzfilter die spezifische Fluoreszenzanregungsstrahlung und lässt die Fluoreszenzemissionsstrahlung entlang des optischen Strahlengangs durch. Bevorzugt blockiert das Fluoreszenzfilter vollständig das Anregungslicht, während es das Fluoreszenzemissionslicht durchlässt, welches üblicherweise eine längere Wellenlänge als das Anregungslicht aufweist. Somit handelt es sich bei einem Fluoreszenzfilter insbesondere um ein Beobachtungsfilter, welches das Anregungslicht, welches ein Fluorophor zum Leuchten bringt, herausfiltert. Dies ist vorteilhaft, da das Anregungslicht in der Regel mehrere Größenordnungen heller ist als das resultierende und/oder emittierte Fluoreszenzlicht und dieses sonst überstrahlt. Bei einem Fluoreszenzfilter kann es sich auch um ein "Blaufilter", "Rotfilter", "IR-Filter" oder "NIR-Filter" handeln.

Unter "Blaufilter" wird insbesondere ein Filter verstanden, welches das blaue Anregungslicht einer Lichtquelle herausfiltert, aber das Fluoreszenzlicht, insbesondere ein von einem Fluorophor emittiertes Fluoreszenzlicht, zumindest überwiegend durchlässt. Beispielsweise erfolgt bei der Verwendung des Fluorophor FITC (Fluorescein isothiocyanat, grünes Derivat von Fluorescein) eine Anregung mittels LED bei einer Wellenlänge von 460 nm, wobei langwelligeres Fluoreszenzlicht mit einem Maximum bei ca. 520 nm im grünen Spektralbereich vom Fluorophor emittiert wird. Um das emittierte Fluoreszenzlicht des FITC in der Bildgebung gut darstellen zu können, wird mittels der Filterwechselvorrichtung und/oder Kamera das blaue Anregungslicht herausgefiltert.

Unter "Rotfilter" wird insbesondere ein Filter verstanden, welches das rote Anregungslicht einer Lichtquelle herausfiltert, aber das Fluoreszenzlicht, insbesondere ein von einem Fluorophor emittierte Fluoreszenzlicht, zumindest überwiegend durchlässt.

Unter "IR-Filter" wird insbesondere ein Filter verstanden, welches infrarotes Anregungslicht einer Lichtquelle herausfiltert, aber das Fluoreszenzlicht, insbesondere ein von einem Fluorophor emittiertes Fluoreszenzlicht, zumindest überwiegend durchlässt.

Unter "NIR-Filter" wird insbesondere ein Filter verstanden, welches nahinfrarotes Anregungslicht einer Lichtquelle herausfiltert, aber das Fluoreszenzlicht, insbesondere ein von einem Fluorophor emittiertes Fluoreszenzlicht, zumindest überwiegend durchlässt.

Eine "Filtermittelachse" ist insbesondere diejenige Achse, welche durch die Mitte der Fläche des optischen Filters geht. Die Filtermittelachse steht insbesondere senkrecht auf der Fläche des optischen Filters. Im Falle eines kreisrunden optischen Filters ist die Filtermittelachse insbesondere in der Mitte des Querschnittes des optischen Filters angeordnet und somit konzentrisch vom kreisrunden Außenumriss des optischen Filters umgeben. Die Filtermittelachse ist insbesondere im Wesentlichen parallel zur Rotationsachse und/oder zur optischen Achse angeordnet.

Ein "optischer Durchgang" ist insbesondere ein ausgesparter Raum in der Filterwechselvorrichtung, durch welchen Licht durchtreten kann. Ein optischer Durchgang ist insbesondere eine durchgehende Öffnung durch das Gehäuse, die Gehäuseteile, das drehbare Antriebsrad und/oder weitere Bestandteile der Filterwechselvorrichtung entlang der optischen Achse. Der optische Durchgang ist insbesondere um den Mittelpunkt des Querschnittes des mindestens einem Gehäuseteil, des drehbaren Antriebsrades und/oder um die optische Achse angeordnet. Der optische Durchgang ist insbesondere konzentrisch um die optische Achse angeordnet. Der optische Durchgang erstreckt sich in Längsrichtung insbesondere entlang der optischen Achse. In Lichtausbreitungsrichtung vor und/oder in dem optischen Durchgang kann insbesondere eine Filteraufnahme und/oder ein optisches Filter angeordnet sein. Ebenso kann der optische Durchgang bei Lichtdurchtritt frei von einem angeordneten optischen Filter und/oder einer Aufnahme sein. Prinzipiell kann der optische Durchgang sämtliche Querschnittsformen aufweisen, bevorzugt ist der optische Durchgang kreisrund im Querschnitt ausgebildet.

Eine "optische Achse" ist insbesondere eine Linie, entlang der ein Grad an Rotationssymmetrie in einem optischen System existiert. Die optische Achse ist insbesondere eine imaginäre Linie, welche einen Pfad definiert, entlang welchem Licht durch die Filterwechselvorrichtung und/oder die Kamera in Richtung eines Bildsensors propagiert. Vorzugsweise läuft die optische Achse durch den Krümmungsmittelpunkt des jeweils eingeschwenkten Filters und/oder eines nachgeschalteten Linsensystems und/oder Objektivsystems. Die optische Achse kann jedoch auch durch eine Linse, ein optisches Element und/oder eines der optischen Filter gebogen und/oder gelenkt werden. Der optische Strahlengang als geometrischer Verlauf von Lichtstrahlen ist insbesondere in und/oder um die optische Achse angeordnet und verläuft entlang, konvergierend und/oder dispergierend zu der optischen Achse.

Prinzipiell ist herauszustellen, dass die Bezeichnungen "erster" und "zweiter" Filterhalterarm, Filter und weitere Begriffe nur der Unterscheidung dienen. So hängt es beispielsweise beim Drehen des Antriebszahnrades von der Drehrichtung im Uhrzeigersinn oder entgegen des Uhrzeigersinns ab, welche Filteraufnahme des zugehörigen Filterhalterarms als erste in den optischen Durchgang eingeschwenkt wird.

Ein "Filterhalterarm" (auch vereinfacht "Filterhalter" genannt) ist insbesondere ein länglich ausgebildetes Element und/oder ein Arm, welcher drehbar um seine Rotationsachse auf und/oder an einer innenliegenden Seite eines Gehäuseteils angeordnet ist. Prinzipiell kann der Filterhalterarm jegliche Form aufweisen. Bevorzugt ist die Filteraufnahme am breitesten Ende des Filterhalterarms angeordnet, wobei in diesem Abschnitt ein Außendurchmesser des Filterhalterarms insbesondere nur geringfügig größer als ein Durchmesser des aufgenommenen optischen Filters ist. Ausgehend von der Filteraufnahme kann der Filterhalterarm sich schrittweise und/oder kontinuierlich in Richtung zur Rotationsachse verjüngen. Beispielsweise kann der Filterhalterarm eine tropfenförmige oder keulenförmige Form aufweisen. Der Filterhalterarm kann in einer Ebene oder abschnittsweise in zwei oder mehreren Ebenen ausgebildet sein. Ebenso kann der Filterhalterarm unterschiedliche Materialstärken aufweisen. Der Filterhalterarm weist insbesondere eine Bohrung an seiner Unterseite auf, wobei in dieser Bohrung die Rotationswelle angeordnet ist. Die Rotationswelle kann insbesondere stoffschlüssig und/oder kraftschlüssig in dieser Bohrung verbunden, beispielsweise eingepresst, sein. Diese Bohrung kann durch eine Materialstärke des Filterhalterarms quer zu seiner Längsrichtung teilweise oder vollständig durchgehend ausgeführt sein. Das andere, der Bohrung gegenüberliegende Ende der Rotationswelle ist insbesondere an dem einen Gehäuseteil verbunden und/oder befestigt. Die Rotationswelle und somit die Rotationsachse des jeweiligen Filterhalterarms ist insbesondere endständig und/oder auf der gegenüberliegenden Seite zur Filteraufnahme des Filterhalterarms entlang der Längsrichtung des Filterhalterarms angeordnet. Prinzipiell können die zwei oder mehreren Filterhalterarme gleiche und/oder unterschiedliche Längen aufweisen. Die Längsrichtung des jeweiligen Filterhalterarms ist insbesondere im Wesentlichen quer zur optischen Achse angeordnet. An der Rotationswelle jedes Filterhalterarms ist insbesondere ein Filterzahnrad verbunden. Bevorzugt umgibt das Filterzahnrad die Rotationswelle. Das jeweilige Filterzahnrad ist insbesondere bewegungsübertragend mit der jeweiligen Rotationswelle verbunden, sodass eine Rotationsbewegung des Filterzahnrades über die verbundene Rotationswelle direkt in eine Rotation des gesamten Filterhalterarms überführt wird. Die Rotationswellen von zwei oder mehreren Filterhalterarmen sind insbesondere parallel zueinander angeordnet. Bevorzugt sind die Rotationswellen und somit die Rotationsachsen gleich beabstandet vom optischen Durchgang an dem ersten Gehäuseteil angeordnet. Die Rotationswellen und/oder Rotationsachsen von zwei oder mehreren Filterhalterarmen können insbesondere im Querschnitt kreisförmig verteilt an dem einen Gehäuseteil angeordnet sein. Die Rotationswellen und/oder Rotationsachsen sind insbesondere derart zu den Zähnen des Antriebsrades angeordnet, dass die Zähne der Filterzahnräder jeweils direkt oder indirekt in die Zähne des drehbaren Antriebszahnrades eingreifen. Vor allem sind die Rotationswellen und/oder -achsen der Filterhalterarme derart angeordnet, dass die Zähne der jeweiligen Filterzahnräder nicht ineinandergreifen können, sondern nur jeweils mit den Zähnen des Antriebszahnrades eingreifen. Bevorzugt sind die Filterhalterarme in ihrer Form, beispielsweise durch Abrundungen und/oder Verengungen, und in ihrer Anordnung derart aufeinander abgestimmt, dass sich die Filterhalterarme bei einer Drehung nicht berühren und möglichst wenig Spiel vorgesehen ist. Bevorzugt ist der jeweilige Filterhalter dazu abgesetzt in zwei ebenen Abschnitten mit unterschiedlichen Höhen und/oder Materialstärken ausgebildet. Dadurch können sich die Filterhalter auch berührungslos zumindest teilweise übereinander und somit überschneidend bewegen. Die jeweilige Form und/oder die Maße eines jeweiligen Filterhalterarms hängen insbesondere vom Durchmesser des optischen Filters und von der optischen Auslegung ab.

Ein "Filterzahnrad" ist insbesondere ein Zahnrad, welches an seinem Außenumfang vollständig umlaufend verteilte Zähne aufweist. Das Filterzahnrad weist insbesondere mittig eine durchgehende Bohrung auf, durch welche die Rotationswelle geführt ist. Ebenso kann aber auch die Rotationswelle des Filterhalterarms lediglich den Filterhalterarm selbst mit dem Filterzahnrad verbinden und nicht vollständig durch das Filterzahnrad bis zum Gehäuseteil durchgehend ausgebildet sein. In diesem Fall weist das Filterzahnrad eine untere Bohrung auf, in welcher beispielweise ein Zylinderstift befestigt an dem Gehäuseteil angeordnet ist, sodass da Filterzahnrad um den Zylinderstift und somit der gesamte Filterhalterarm drehbar ist.

Eine "Rotationsachse" ist insbesondere eine Achse, mit welcher der Filterhalterarm verbunden ist und um welche der Filterhalterarm rotiert. Die Rotationsachse ist insbesondere diejenige Gerade, welche bei einer Drehbewegung eines Filterhalterarms ortsfest bleibt. Die Rotationsachse ist insbesondere eine Längsmittelachse der Rotationswelle. Die Rotationsachse ist insbesondere parallel zur optischen Achse.

Eine "Filteraufnahme" (auch "Aufnahme" genannt) ist insbesondere ein Hohlkörper oder Hohlraum mit einer außen teilweisen oder vollständigen Umfassung und/oder Umrandung, in welchen ein optisches Filter einsetzbar ist und welche den optischen Filter an seinem Umfang zumindest teilweise umschließt und hält. Eine Filteraufnahme kann beispielsweise ein kurzer rohrförmiger Körper sein. Die Filteraufnahme bildet insbesondere einen Halter und/oder eine Schutzhülle für das optische Filter. Eine Filteraufnahme kann beispielsweise auch einen Federring zum Halten des optischen Filters aufweisen.

Ein "Antriebszahnrad" ist insbesondere eine Scheibe oder ein Ring, welche oder welcher mindestens eine umlaufende Verzahnung aufweist. Das Antriebszahnrad ist insbesondere ein ebenes, flaches Bauteil, wobei dessen gegenüberliegende Flächen im Wesentlichen senkrecht zur optischen Achse und/oder zum optischen Durchgang ausgerichtet sind. Das Antriebszahnrad weist insbesondere eine Außenverzahnung und/oder eine Innenverzahnung auf. Das Antriebszahnrad kann insbesondere auch als Antriebsrad ausgebildet sein und somit von außen insbesondere per Hand, mittels einer Antriebseinheit und/oder einem Motor angetrieben und gedreht werden. Dazu kann beispielsweise eine äußere Umfangsfläche des Antriebszahnrads als Kontaktfläche ausgebildet sein und angetrieben werden, indem eine Antriebseinheit und/oder eine Getriebe an dieser und/oder auf diese Kontaktfläche einwirkt. Als Kontaktfläche kann das Antriebszahnrad beispielsweise eine Außenverzahnung aufweisen, in welche ein Antrieb von außen eingreift. Neben einer optionalen Verzahnung zum Antreiben des Antriebszahnrades selbst weist das Antriebszahnrad in dieser Ausführungsform insbesondere eine rundumlaufende Innenverzahnung zum Übertragen seiner Drehbewegung auf die Filterzahnräder auf. Eine innenliegende Innenverzahnung des Antriebsrades ist insbesondere konzentrisch um den optischen Durchgang angeordnet. Somit bildet ein Raum zwischen dem optischen Durchgang und der Innenverzahnung des Antriebsrades insbesondere einen Raum zur Aufnahme der Filterzahnräder der Filterhalterarme aus. Je nachdem, ob die Innen- oder Außenverzahnung des Antriebszahnrades direkt oder indirekt in die Filterzahnräder eingreift, sind die jeweiligen Rotationswellen und/oder Zylinderstifte der jeweiligen Filterhalterarme in einem definierten Abstand von der Innen- oder Außenverzahnung des Antriebszahnrades angeordnet. Im Falle, dass das Antriebszahnrad mit einer Außenverzahnung zum Antreiben der Filterzahnräder ausgebildet ist, ist das Antriebszahnrad insbesondere um den optischen Durchgang angeordnet. In diesem Fall kann das Antriebszahnrad insbesondere an einem Filterrad befestigt sein, dessen Außenumfangsfläche insbesondere zum Antreiben dient. Entsprechend einer Ausbildung des Antriebszahnrades mit einer Außenverzahnung zum Eingreifen in die Filterzahnräder der Filterhalterarme sind die Rotationswellen der Filterhalterarme beabstandet außen um die Außenverzahnung des Antriebsrades angeordnet. Folglich sind in diesem Falle die Filterhalterarme weiter vom optischen Durchgang beabstandet. Demgegenüber weist die Ausbildungsform des Antriebsrades mit einer Innenverzahnung zum Eingreifen in die Filterzahnräder den Vorteil auf, dass die Filterhalterarme näher an dem optischen Durchgang angeordnet sind und somit schneller in den optischen Durchgang eingeschwenkt werden können. Zudem kann bei einer Ausbildung des Antriebszahnrades mit einer antreibenden Innenverzahnung das gesamte Antriebszahnrad als Antriebs- und/oder Filterrad ausgebildet sein und direkt an seinem Außenumfang angetrieben werden. Folglich weist die Ausbildungsform des Antriebszahnrades mit einer Innenverzahnung zum Antreiben der Filterzahnräder eine geringere Baugröße auf. Das Antriebszahnrad ist insbesondere zwischen den beiden Gehäuseteilen drehbar haltbar und dadurch gelagert. Das Antriebszahnrad ist insbesondere unendlich und somit mit einer beliebigen Anzahl von Umdrehungen im Uhrzeigersinn und/oder gegen den Uhrzeigersinn drehbar. Dementsprechend sind durch die Übertragung mittels des jeweiligen Filterzahnrades und der Rotationswelle auch die Filterhalterarme beliebig unendlich in jede der beiden Drehrichtungen drehbar. Um die Reibungskräfte beim Eingreifen der jeweiligen Zähne zu minimieren, können die Filterzahnräder und das Antriebszahnrad insbesondere ein Material mit einem niedrigen Reibungskoeffizienten, beispielsweise Aluminium und/oder ein polymeres Material, wie PTFE, aufweisen.

Eine "Gehäuseteil" ist insbesondere ein Bestandteil der Filterwechselvorrichtung, auf und/oder an welchem die mindestens zwei Filterhalterarme drehbar befestigt sind. Bei dem Gehäuseteil kann es sich um ein distales oder proximales Gehäuseteil und/oder um einen distalen oder proximalen Gehäusedeckel der Filterwechselvorrichtung handeln. Bei dem Gehäuseteil kann es sich auch um eine Grundplatte innenliegend im Gehäuse handeln. Ebenso kann eine Grundplatte zum Zwischenlagern und Durchführen der Rotationswellen angeordnet sein. Die beiden Gehäuseteile sind insbesondere derart miteinander verbunden, dass das dazwischen angeordnete Antriebszahnrad und/oder Antriebsrad frei drehbar ist. Dazu kann beispielweise mindestens ein zur Umfangfläche des Antriebszahnrades beabstandeter Steg in einer Richtung parallel zur optischen Achse die beiden Gehäuseteile verbinden, wobei das freie Ende des Stegs beispielsweise mittels einer Schraubverbindung am anderen Gehäuseteil befestigt sein kann. Ebenso können die beiden Gehäuseteile im Inneren des Gehäuses miteinander verbunden sein. Damit kann das Antriebszahnrad und/oder Antriebsrad auch einen größeren Durchmesser aufweisen als ein Gehäuseteil oder beide Gehäuseteile.

Eine "Kamera" (auch "Kamerakopf" genannt) ist insbesondere ein Gerät zum Empfangen von Bildlicht entlang einer optischen Achse von einem Endoskop und zum Fokussieren des empfangenen Bildlichtes auf mindestens einem Bildsensor. Neben dem mindestens einen Bildsensor kann die Kamera insbesondere eine Blende oder ein Fenster zum Durchlassen des empfangenen Bildlichtes und ein Linsensystem zum Fokussieren des Bildlichtes auf den mindestens einen Bildsensor aufweisen. Die durch mindestens einen Bildsensor aufgenommenen Bilddaten sind insbesondere elektronisch vom Kamerakopf weiter an ein Anzeigesystem und/oder an eine Bildverarbeitungseinheit übertragbar, um das endoskopische Bild für den Benutzer darzustellen. Die Kamera kann Mittel zum Erkennen des verbundenen Endoskopes und zum Verarbeiten von Algorithmen aufweisen. Ein Verbinder zum Verbinden eines Endoskops mit der Kamera kann am distalen Ende des Endoskops und/oder dem proximalen Ende des Kamerakopfs angeordnet sein. Auch kann die erfindungsgemäße Filterwechselvorrichtung selbst als Verbinder zum Verbinden eines Endoskopes mit einer Kamera ausgebildet sein.

Ein "Endoskop" ist insbesondere ein medizinisches oder industrielles Gerät zur endoskopischen Untersuchung und Betrachtung einer menschlichen oder tierischen Körperhöhle und/oder eines industriellen Hohlraums, wie einem Rohr. Das Endoskop weist insbesondere ein Handstück, einen Schaft, eine Lichtquelle, einen Lichtleiter, einen Sensor und/oder eine Kamera auf. Bei dem Endoskop handelt es sich insbesondere um ein Videoendoskop, welches eine digitale Bildaufnahme und Bildübertragung und somit eine integrierte oder verbindbare Kamera aufweist. Neben human- und tiermedizinischen Anwendungen kann ein Endoskop und/oder Videoendoskop auch zu industriellen Zwecken, beispielsweise zur Sichtprüfung in schwer zugänglichen Hohlräumen, eingesetzt werden. Bei industriellen Anwendungen wird ein Endoskop häufig als Boroskop bezeichnet.

Ein "Bildsensor" ist insbesondere ein lichtempfindliches elektronisches Bauelement, welches auf einem inneren Photoeffekt beruht. Mittels des Bildsensors wird insbesondere ein Bild oder werden mehrere Bilder aus dem Betrachtungsbereich der Bildgebungsvorrichtung aufgezeichnet und in elektronische Signale umgewandelt. Der Bildsensor weist eine Sensorebene in der Bildebene des optischen Systems, eines Linsensystems und/oder eines Objektivs auf. Bei einem elektronischen Bildsensor kann es sich insbesondere um einen CCD-Sensor (charge-coupled device) oder um einen CMOS-Sensor (complementary metal oxide-semiconductor) handeln.

Unter "distalseitig" und "distal" wird insbesondere eine benutzerferne Anordnung und/oder ein entsprechendes Ende oder ein Abschnitt verstanden. Dementsprechend ist bei einem Verbinden der Filterwechselvorrichtung mit einem Endoskop das Endoskop distalseitig angeordnet. Dementsprechend wird unter "proximalseitig" oder "proximal" eine benutzernahe Anordnung oder ein entsprechendes Ende oder ein Abschnitt verstanden. Bei Verbindung der Filterwechselvorrichtung mit einer Kamera und/oder einem Kamerakopf ist entsprechend die Kamera und/oder der Kamerakopf proximalseitig von der Filterwechselvorrichtung angeordnet.

In einer weiteren Ausführungsform weist die Filterwechselvorrichtung einen dritten Filterhalterarm, einen vierten Filterhalterarm, einen fünften Filterhalterarm und/oder optional weitere Filterhalterarme auf.

Dadurch können weitere unterschiedliche Filter oder kombiniert gleiche und unterschiedliche Filter in den optischen Strahlengang beim Drehen des Antriebszahnrades ein- und ausgeschwenkt werden. Entsprechend der gewünschten Häufigkeit in der Anwendung kann beispielsweise ein häufig genutztes Filter mehrmals bei einer Umdrehung des Antriebsfilterzahnrades in den optischen Durchgang ein- und wieder ausgeschwenkt werden. Bei Verendung von fünf Filterarmen können beispielsweise drei Fluoreszenzfilter, ein Weißlichtfilter und ein leerer Filterplatz für multispektrale Bildgebung jeweils in einer Filteraufnahme der fünf Filterhalterarme angeordnet sein. Ebenso können sich aber auch gleiche oder unterschiedliche Fluoreszenzfilter jeweils mit einem nachfolgenden Weißlichtfilter abwechseln.

Dadurch, dass die Filterwechselvorrichtung nur durch ein einziges Antriebszahnrad angetrieben und die Filterzahnräder dadurch bewegt werden, kann aufgrund des Innen- oder Außendurchmessers des Antriebszahnrades und somit der Länge der rundumlaufenden Verzahnung in einfacher Weise die Filterwechselvorrichtung auf eine unterschiedliche Anzahl von Filterhaltearmen skaliert und realisiert werden. Folglich lässt sich die Filterwechselvorrichtung und deren Antrieb weitgehend unabhängig von der Anzahl der Filterhalterarme in einfacher Weise und zuverlässig automatisieren.

Um eine kompakte Baugröße der Filterwechselvorrichtung und kollisionsfreie Bewegungen der Filterhalterarme zu gewährleisten, kann ein Abstand zwischen der Filtermittelachse und der Rotationsachse eines Filterhalterarms das 0,80-Fache bis 1,10-Fache eines maximalen Außendurchmessers des Filterhalterarms im Bereich der Filteraufnahme sein.

In einer weiteren Ausführungsform der Filterwechselvorrichtung ist ein Abstand zwischen zwei Rotationsachsen zweier Filterhalterarme das 1,05-fache bis 1,50-fache eines maximalen Außendurchmessers des jeweiligen Filterhalterarms im Bereich der Filteraufnahme.

Somit ist der Abstand der Drehachsen zweier benachbarter Filterhalterarme nur geringfügig größer als der maximale Außendurchmesser des jeweiligen Filterhalterarms. Folglich ist der durch das Antriebszahnrad und den optischen Durchgang vorgegebene zur Verfügung stehende Raum zum Anordnen und/oder Bewegen der Filterhalterarme optimal ausnutzbar.

Damit ein Filterhalterarm zumindest teilweise über und/oder unter einen benachbarten anderen Filterhalterarm schiebbar und/oder bewegbar ist, kann der jeweilige Filterhalterarm einen ersten Armabschnitt mit der angeordneten Rotationswelle und einen zweiten Armabschnitt mit der angeordneten Filteraufnahme aufweisen, wobei beide Armabschnitte mittels eines Übergangsabschnittes verbunden sind.

Somit können die beiden Armabschnitte des jeweiligen Filterhalterarms auf unterschiedlichen Höhen des Filterhalterarms in einer Richtung entlang der Rotationsachse und somit mit unterschiedlichen Abständen zu dem einen Gehäuseteil angeordnet sein.

Der "erste Armabschnitt" und der "zweite Armabschnitt" sind insbesondere in Längsrichtung des Filterhalterarms und/oder entlang der Rotationsachse unterschiedlich ausgebildet. Die beiden Armabschnitte können insbesondere unterschiedliche Formen, Abrundungen und/oder Materialstärken aufweisen. Beispielsweise kann der erste Armabschnitt mit der angeordneten Rotationswelle im Wesentlichen oval im Querschnitt quer zur Rotationsachse ausgebildet sein und der zweite Armabschnitt mit der Filteraufnahme kann kreisrund ausgebildet sein. Bevorzugt weist der zweite Armabschnitt mit der Filteraufnahme einen größeren Durchmesser als der erste Armabschnitt auf. Unter einer unterschiedlichen Höhe wird auch verstanden, dass bei angeordneten Rotationswellen am distalen Gehäuseteil eine proximale Fläche eines Armabschnittes weiter in der proximalen Richtung angeordnet ist als eine proximale Fläche des anderen Armabschnittes. Ebenso kann bei angeordneten Rotationswellen am proximalen Gehäuseteil eine distale Fläche einer Armabschnittes weiter in der distalen Richtung angeordnet sein als die distale Fläche des anderen Armabschnittes.

Ein "Übergangsabschnitt" ist insbesondere ein Abschnitt zwischen dem ersten Armabschnitt und dem zweiten Armabschnitt, welcher beide Armabschnitte verbindet und/oder die unterschiedlichen Eigenschaften beider Armabschnitte im Übergang aneinander angleicht. Bei dem Übergangsabschnitt kann es sich insbesondere um einen geformten und/oder gebogenen Abschnitt des Filterhalterarms handeln, welcher beispielsweise die unterschiedlichen Höhen des ersten Armabschnittes und des zweiten Armabschnittes verbindet. Prinzipiell ist herauszustellen, dass der Filterhalterarm mit seinen Abschnitten mehrteilig, verbunden oder einstückig ausgebildet sein kann. Beispielsweise kann der Filterhalterarm als ein Bauteil gegossen gefertigt sein. Ebenso können beispielsweise auch die beiden Armabschnitte und der Übergangsabschnitt jeweils stoffschlüssig, beispielsweise durch Verkleben, verbunden sein.

In einer weiteren Ausführungsform der Filterwechselvorrichtung sind der erste Armabschnitt und der zweite Armabschnitt in einer Richtung entlang der Rotationsachse zueinander beabstandet ausgebildet.

Somit sind der erste Armabschnitt und der zweite Armabschnitt zueinander abgesetzt und/oder auf unterschiedlichen Höhen beabstandet vom Gehäuseteil angeordnet.

Prinzipiell ist herauszustellen, dass sowohl der erste Armabschnitt näher entlang der Rotationsachse an dem Gehäuseteil als der zweite Armabschnitt angeordnet sein kann oder umgekehrt. Bevorzugt ist aufgrund der Rotationsachse jedoch der erste Armabschnitt näher an dem Gehäuseteil und somit der zweite Armabschnitt höher und somit mit einem größeren Abstand zum Gehäuseteil angeordnet.

Damit zwei benachbarte Filterarme zumindest teilweise sich auf überschneidenden Kreisradien bewegen können, kann der zweite Armabschnitt in einer Richtung entlang der Rotationsachse auf einer größeren Höhe als eine Höhe des ersten Armabschnittes angeordnet sein, sodass ein zweiter Armabschnitt eines ersten Filterhalterarms zumindest teilweise über einen ersten Armabschnitt eines zweiten Filterhalterarms anordenbar und/oder drehbar ist.

Dadurch können mehrere Filterhalterarme kompakter im zur Verfügung stehenden Bauraum beispielsweise zwischen dem optischen Durchgang und dem Antriebszahnrad angeordnet sein und/oder die Anzahl der Filterhalterarme kann in einfacher Weise ohne weitere Modifikationen der Filterwechselvorrichtung in einer bestehenden Filterwechselvorrichtung erhöht werden.

In einer weiteren Ausführungsform der Filterwechselvorrichtung ist eine Differenz zwischen einer maximalen Höhe des zweiten Armabschnittes und einer maximalen Höhe des ersten Armabschnittes ein 1,00-Faches bis 1,50-Faches einer maximalen Materialstärke des zweiten Armabschnittes in eine Richtung entlang der Rotationsachse.

Zuzüglich möglicher Fertigungstoleranzen liegt somit entlang der Rotationsachse ein ausreichender Freiraum zwischen einer Unterseite des zweiten Armabschnittes mit der Filteraufnahme eines ersten Filterhalterarms und der Oberseite des ersten Armabschnittes eines zweiten Filterhalterarms bei senkrechter Anordnung der Rotrationsachse vor.

Um die jeweilige Rotationswelle axial zu sichern, kann die jeweilige Rotationswelle mittels eines Sicherungselementes an dem mindestens einen Gehäuseteil verbunden sein.

Bei einem "Sicherungselement" kann es sich beispielsweise um einen Stellring oder einen Axialsicherungsring handeln.

In einer weiteren Ausführungsform der Filterwechselvorrichtung weist das Antriebszahnrad eine Innenverzahnung und/oder eine Außenverzahnung auf.

Weist das Antriebszahnrad sowohl eine Innenverzahnung und eine Außenverzahnung auf, so kann die Außenverzahnung für den Antrieb des Antriebszahnrades mittels eines Getriebes und/oder Motors und die Innenverzahnung zum Antrieb der Filterzahnräder genutzt werden. Prinzipiell kann das Antriebszahnrad auch mit einem Antriebsrad verbunden und/oder mit diesem kombiniert werden. Hierzu kann beispielsweise das Antriebszahnrad um den optischen Durchgang der Filterwechselvorrichtung mit einer Außenverzahnung zum Antreiben der Filterzahnräder auf einer distalen oder proximalen Fläche eines Antriebsrades angeordnet sein und das Antriebsrad an seinem Außendurchmesser selbst eine Außenverzahnung zum Antreiben mittels eines Getriebes und/oder Motors aufweisen.

Um das Antriebszahnrad in einfacher Weise zu lagern und drehbar auszubilden, kann die Filterwechselvorrichtung ein zweites Gehäuseteil aufweisen und das erste Gehäuseteil und das zweite Gehäuseteil können in ihrem Inneren und/oder an ihrem Äußeren miteinander verbunden sein, sodass das Antriebszahnrad über seinen gesamten Umfang von außen frei antreibbar und/oder drehbar ist.

Dadurch kann das Antriebszahnrad frei drehbar und sein Außenumfang frei kontaktierbar sein.

In einer weiteren Ausführungsform weist die Filterwechselvorrichtung einen Motor zum Antreiben des drehbaren Antriebszahnrades auf.

In einem weiteren Aspekt der Erfindung wird die Aufgabe gelöst durch einen Kamerakopf für ein Endoskop, wobei der Kamerakopf einen Bildsensor, eine Öffnung zum Empfangen von Licht eines Bildes entlang eines optischen Pfades und ein optisches Linsensystem zum Fokussieren des Lichtes auf den Bildsensor aufweist, wobei der Kamerakopf zumindest eine zuvor beschriebene Filterwechselvorrichtung aufweist.

Somit wird ein Kamerakopf bereitgestellt, an dem oder in dem eine kompakte, platzsparend ausgebildete Filterwechselvorrichtung angeordnet ist, welche einen schnellen Filterwechsel und ein schnelles Durchrotieren von verschiedenen Filtern hintereinander ermöglicht. Im Falle der Anordnung zumindest einer Filterwechselvorrichtung direkt in dem Kamerakopf kann der Kamerakopf lösbar mit verschiedenen Arten von Endoskopen verbunden werden. Beispielsweise kann die Filterwechselvorrichtung in dem Kamerakopf integriert werden, indem diese anstelle eines Bajonett-Verschlusses verbaut ist. Dadurch kann eine Verlängerung des Strahlenganges minimiert werden. Selbstverständlich kann der Kamerakopf auch zwei oder mehrere Filterwechselvorrichtungen in Reihe in einem optischen Pfad und/oder entlang der optischen Achse aufweisen, falls die gleichzeitige Anwendung von zwei oder mehreren Filtern im Strahlengang, insbesondere in multispektralen Bildgebungen und/oder in einer breiten Anwendung von verschiedenen Fluorophoren in der Fluoreszenz-Bildgebung, gewünscht ist.

Um eine derzeitig verwendete Filterkonfiguration oder diese für gewünschte unterschiedliche Beobachtungsmodi anzupassen, kann der Kamerakopf eine Detektionseinheit zum Detektieren einer Identifikation des jeweiligen optischen Filters in dem optischen Pfad aufweisen. Ebenso kann der Kamerakopf eine Kontrolleinheit zum Anpassen der Drehgeschwindigkeit des Antriebszahnrades und zum Überprüfen und/oder Anpassen des jeweiligen optischen Filters angeordnet im Strahlengang entsprechend des jeweils gewählten Betriebsmodus aufweisen.

In einem weiteren Aspekt der Erfindung wird die Aufgabe gelöst durch einen Nachrüstsatz zum Nachrüsten eines Kamerakopfes und/oder eines Endoskopes, wobei der Nachrüstsatz zumindest eine zuvor beschriebene Filterwechselvorrichtung aufweist, sodass die Filterwechselvorrichtung zwischen einem proximalen Ende des Endoskops und einem distalen Ende des Kamerakopfes anordenbar ist.

Somit wird ein Nachrüstsatz (auch als "Adapter" bezeichnet) mit mindestens einer Filterwechselvorrichtung bereitgestellt, welcher gleichzeitig als Verbinder zwischen einem bereits existierenden Endoskop und einem existierenden Kamerakopf als auch zum Ermöglichen von unterschiedlichen Beobachtungsmodi dient. Zusätzlich kann der Nachrüstsatz auch zwei oder mehrere Filterwechselvorrichtungen aufweisen, welche in Reihe zwischen dem Endoskop und dem Kamerakopf angeordnet sind oder eine Filterwechselvorrichtung kann durch eine andere Filterwechselvorrichtung zum Ermöglichen von verschiedenen Anwendungen und/oder Beobachtungsoptionen ersetzt werden.

Im Weiteren wird die Erfindung anhand von Ausführungsbeispielen erläutert. Es zeigen
Figur 1 eine schematische dreidimensionale Ausschnittsansicht eines Endoskopsystems mit einem Endoskop, einem Filterwechsler und einem Kamerakopf,
Figur 2 eine dreidimensionale Darstellung eines Filterwechslers in Seitenansicht mit einer Endoskopaufnahme und einer Kameraaufnahme,
Figur 3 eine schematische Ansicht auf eine distale Deckelhälfte des Filterwechslers aus Figur 2,
Figur 4 eine schematische Ansicht auf eine proximale Deckelhälfte des Filterwechslers,
Figur 5 eine dreidimensionale Darstellung des Filterwechslers im geöffneten Zustand mit Sicht auf fünf Filterhalterarme und ein Antriebszahnrad,
Figur 6 eine dreidimensionale Darstellung des Filterwechslers in geöffnetem Zustand aus Figur 5 in Seitenansicht,
Figur 7 eine dreidimensionale Darstellung eines Filterhalterarms in Seitenansicht,
Figur 8 eine Draufsicht auf den Filterhalterarm aus Figur 7, und
Figur 9 eine dreidimensionale Darstellung des Filterwechslers im Querschnitt.

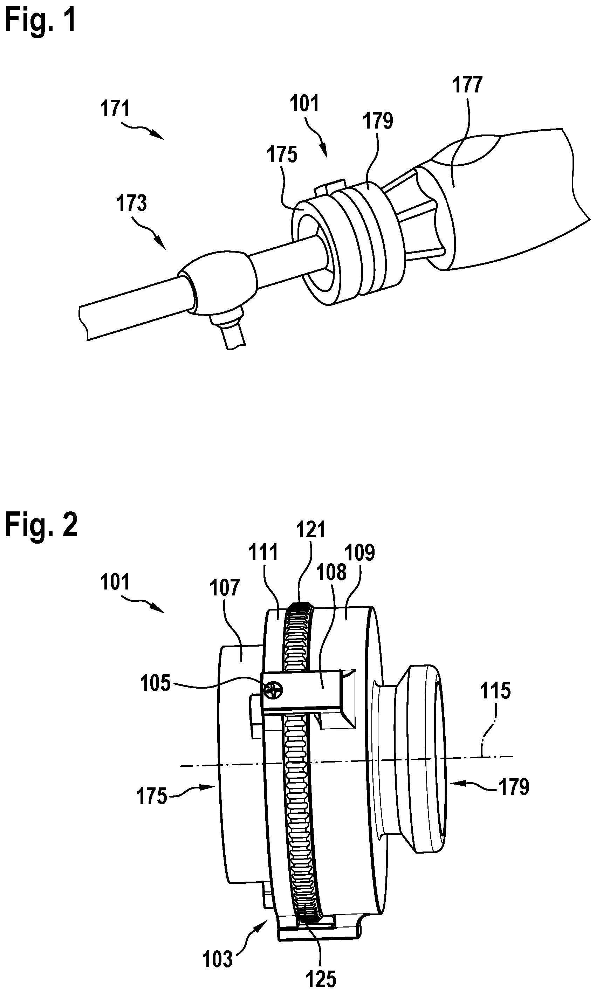

Ein Endoskopsystem 171 weist einen Kamerakopf 177, einen Filterwechsler 101 und ein Endoskop 173 auf. Der Filterwechsler 101 ist mittels einer Kameraaufnahme 179 an dem Kamerakopf 177 und mittels einer Endoskopaufnahme 175 mit dem Endoskop 173 verbunden (Figur 1 und 2).

Der Filterwechsler 101 weist ein Gehäuse 103 mit einer distalen Deckelhälfte 107 mit einer Grundplatte 111 und einer proximalen Deckelhälfte 109 auf, wobei zwischen der Grundplatte 111 und der proximalen Deckelhälfte 109 ein Antriebszahnrad 121 drehbar angeordnet ist. Die proximale Deckelhälfte 109 weist an ihrem Außenumfang drei gleichmäßig verteilte Verbindungsstege 108 auf, welche in ihrer Längsrichtung entlang einer optischen Achse 115 mit einem Abstand zu einem Außendurchmesser des Antriebszahnrades 121 bis zum Außenumfang der Grundplatte 111 geführt sind und dort mittels nach innen gerichteter Schrauben 105 jeweils befestigt sind. Dadurch ist das Antriebszahnrad 121 zwischen der Grundplatte 111 und der proximalen Deckelhälfte 109 drehbar gelagert und rundumlaufend an seiner Außenverzahnung 125 antreibbar (Figur 2).

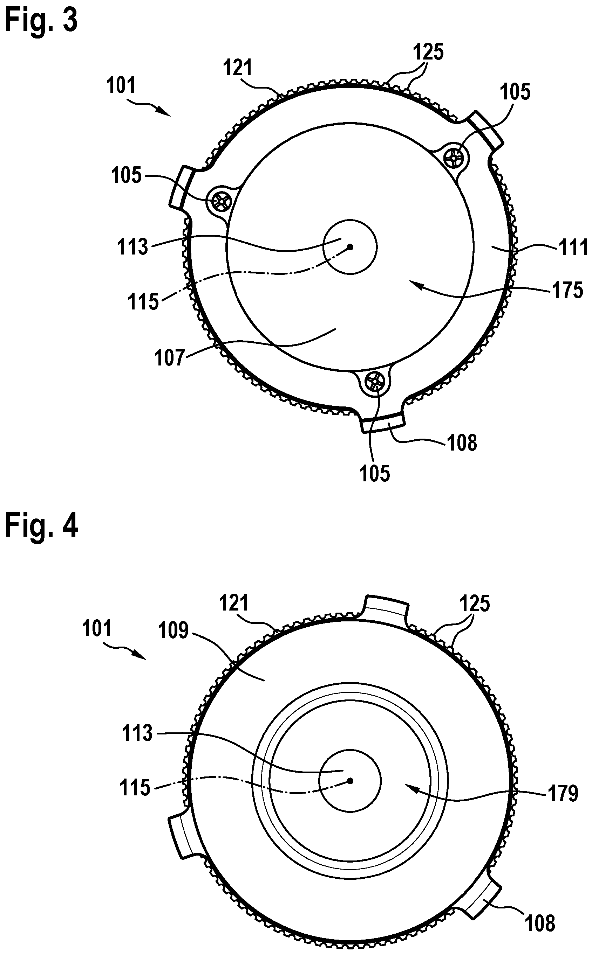

Die distale Deckelhälfte 107 weist distalseitig die Endoskopaufnahme 175 und die proximale Deckelhälfte 109 weist proximalseitig die Kameraaufnahme 179 auf (Figur 3). Die distale Deckelhälfte 107 und die proximale Deckelhälfte 109 weisen jeweils einen optischen Durchgang 113 konzentrisch zur optischen Achse 115 auf. Ebenso weist die Grundplatte 111 einen optischen Durchgang 113 auf.

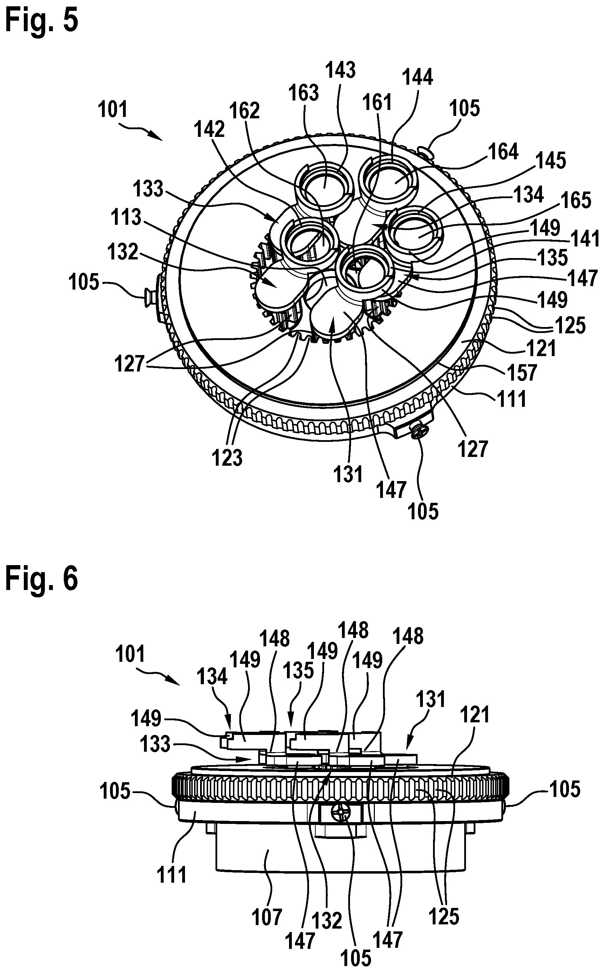

In Figur 5 ist der Filterwechsler 105 bei entfernter proximaler Deckelhälfte 109 gezeigt. In einem Raum zwischen einer Innenverzahnung 123 des Antriebszahnrades 121 und dem optischen Durchgang 113 sind ein erster Filterhalterarm 131 mit einer ersten Filteraufnahme 141 und einem aufgenommenen ersten Filter 161, ein zweiter Filterhalterarm 132 mit einer zweiten Filteraufnahme 142 und aufgenommenem zweiten Filter 162, ein dritter Filterhalterarm 133 mit einer dritten Filteraufnahme 143 und einem aufgenommenen dritten Filter 163, ein vierter Filterhalterarm 134 mit einer vierten Filteraufnahme 144 und einem aufgenommenen vierten Filter 164 und ein fünfter Filterhalterarm 135 mit einer fünften Filteraufnahme 145 und einem aufgenommenen fünften Filter 165 angeordnet. Die Filterhalterarme 131 bis 135 sind innerhalb eines Bewegungsumkreises 175 rotierbar und bewegbar.

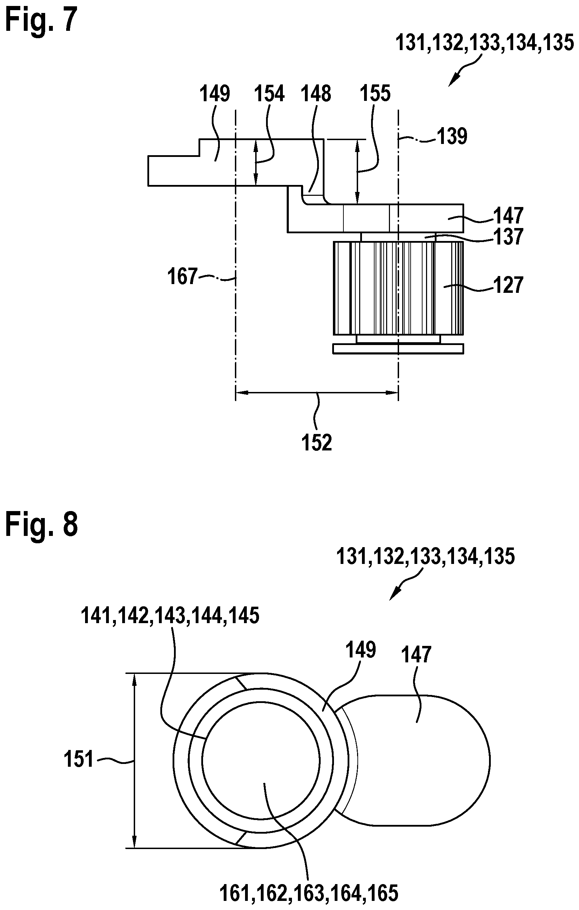

Jeder Filterhalterarm 131 bis 135 ist abgesetzt mit einem ersten Armabschnitt 147 gefolgt von einem Übergangsabschnitt 148 und einem zweiten Armabschnitt 149 ausgebildet (Figuren 7 und 8). Der erste Armabschnitt 147 ist im Querschnitt ovalförmig ausgebildet und an seiner Unterseite mit einer Rotationswelle 137 verbunden. Um die Rotationswelle 137 ist ein Filterzahnrad 127 befestigt. Die Rotationswelle 137 weist eine Rotationsachse 139 auf, welche senkrecht zur Längsausrichtung des jeweiligen Filterhalterarms 131 bis 135 steht. Der zweite Armabschnitt 149 ist in Draufsicht kreisrund ausgebildet, wobei in der jeweiligen Filteraufnahme 141 bis 145 jeweils ein Filter 161, 162, 163, 164, 165 aufgenommen ist.

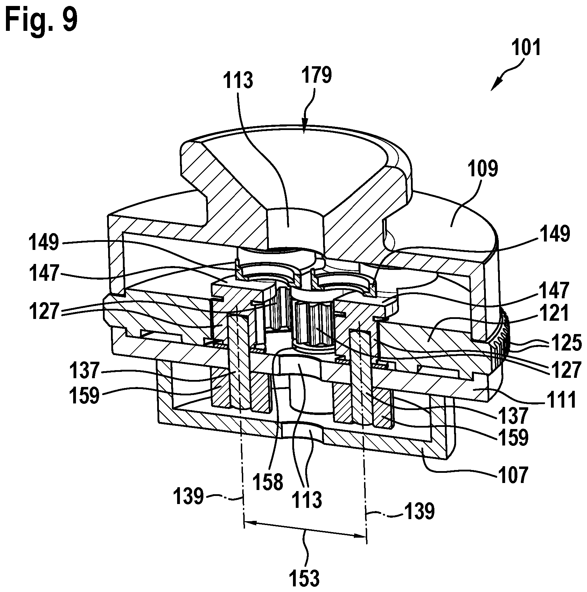

Ein maximaler Außendurchmesser 151 des zweiten Armabschnittes 149 beträgt 9,4 mm und ist größer als ein maximale Außendurchmesser des ersten Armabschnittes 147. Die maximale Materialstärke 154 des zweiten Armabschnittes 149 beträgt 2,5 mm und eine Absatzhöhe 155 zwischen einer Oberseite des zweiten Armabschnittes 149 und einer Oberseite des ersten Armabschnittes 147 beträgt 3,5 mm, sodass zwischen der Unterseite eines zweiten Armabschnittes 149 eines Filterhalterarms und der Oberseite eines ersten Armabschnittes 147 eines daneben angeordneten zweiten Filterhalterarms ein Abstand von 1,0 mm besteht. Ein Abstand 152 zwischen einer Filtermittelachse 167 des jeweils aufgenommenen Filters 161 bis 165 zu der jeweiligen Rotationsachse 139 beträgt 8,8 mm. Die jeweilige Rotationsachse 139 ist durch eine Unterlegscheibe 158 und die Grundplatte 111 geführt und jeweils auf der Innenseite innenliegend an der distalen Deckelhälfte 107 mittels eines Stellrings 159 axial gesichert (siehe Figur 9). Ein Abstand 153 zwischen zwei benachbarten Rotationsachsen 139 beträgt jeweils 10,35 mm. Die jeweiligen Rotationsachsen 139 aller fünf Filterhalter 131 bis 135 sind derart beabstandet zu der Innenverzahnung 123 des Antriebszahnrades 121 angeordnet, dass die Zähne des jeweiligen Filterzahnrades 127 direkt in die Innenverzahnung 123 des Antriebszahnrades 121 eingreifen.

Mit dem Filterwechsler 101 und dem Endoskopsystem 171 werden folgende Arbeitsgänge durchgeführt.

Mittels eines nicht gezeigten Motors und Getrieberades wird das Antriebszahnrad 121 an seiner Außenverzahnung 125 angetrieben und in Drehung im Uhrzeigersinn versetzt. Aufgrund des Eingreifens der Innenverzahnung 123 des Antriebsrads 121 in das jeweilige Filterzahnrad 127 der fünf Filterhalterarme 131, 132, 133, 134, 135 werden diese Filterhalterarme aufgrund der Übertragung der Rotationsbewegung des jeweiligen Filterzahnrad 127 über die jeweilige Rotationswelle 137 in Kreisbewegungen versetzt und bewegen sich entlang der Innenverzahnung 123 im Uhrzeigersinn, wobei der jeweilige zweite Armabschnitt 149 eine entsprechende Kreisbewegung ebenfalls im Uhrzeigersinn durchführt.

In dem in der Figur 5 gezeigten Zustand befindet sich der erste Filterhalterarm 131 in einer Ausschwenkbewegung aus dem optischen Durchgang 113 und aufgrund der Absatzhöhe 155 schwenkt der zweite Armabschnitt 149 des ersten Filterhalterarms 131 mit der ersten Filteraufnahme 141 und dem ersten Filter 161 berührungsfrei über den ersten Armabschnitt 147 des fünften Filterhalters 135. Bei einem weiteren Drehen des Antriebsrades 121 im Uhrzeigersinn wird der zweite Filterhalterarm 132 in den optischen Durchgang 113 eingeschwenkt und der erste Filterhalterarm 131 bewegt sich zeitgleich weiter in Richtung nach außen auf den Bewegungsumkreis 157 zu. Der dritte Filterhalterarm 133, der vierte Filterhalterarm 134 und der fünfte Filterhalterarm 135 bewegen sich entsprechend analog weiter.

Somit wird ein Filterwechsler 101 bereitgestellt, mit welchem aufgrund der räumlich nahen Anordnung und der simultanen Bewegung der Filterhalterarme 131 bis 135 innenliegend zur Innenverzahnung 123 verschiedene Filter 161 bis 165 schnell in den optischen Durchgang 113 nacheinander einschwenkbar sind, wobei jederzeit die Drehrichtung des Antriebsrades 121 vom Anwender gewechselt werden kann. Je nach Wahl der Filter 161 bis 165, beispielsweise drei Fluoreszenzfilter, ein Weißlichtfilter und optional auch eine leere Filteraufnahme für eine multispektrale Beobachtung, kann der Anwender schnell und einfach verschiedene Beobachtungsmodi realisieren und den Filterwechsler 101 mit verschiedenen Arten von Endoskopen 173 und Kameraköpfen 177 einsetzen.

### Bezugszeichenliste

- 101: Filterwechsler
- 103: Gehäuse
- 105: Schraube
- 107: distale Deckelhälfte
- 108: Verbindungssteg
- 109: proximale Deckelhälfte
- 111: Grundplatte
- 113: optischer Durchgang
- 115: optische Achse
- 121: Antriebszahnrad
- 123: Innenverzahnung
- 125: Außenverzahnung
- 127: Filterzahnrad
- 131: erster Filterhalterarm
- 132: zweiter Filterhalterarm
- 133: dritter Filterhalterarm
- 134: vierter Filterhalterarm
- 135: fünfter Filterhalterarm
- 137: Rotationswelle
- 139: Rotationsachse
- 141: erste Filteraufnahme
- 142: zweite Filteraufnahme
- 143: dritte Filteraufnahme
- 144: vierte Filteraufnahme
- 145: fünfte Filteraufnahme
- 147: erster Armabschnitt
- 148: Übergangsabschnitt
- 149: zweiter Armabschnitt
- 151: maximaler Außendurchmesser des zweiten Armabschnittes
- 152: Abstand zwischen Filtermittelachse und Rotationsachse
- 153: Abstand zwischen zwei Rotationsachsen
- 154: maximale Materialstärke des zweiten Armabschnittes
- 155: Absatzhöhe
- 157: Bewegungsumkreis
- 158: Unterlegscheibe
- 159: Stellring
- 161: erster Filter
- 162: zweiter Filter
- 163: dritter Filter
- 164: vierter Filter
- 165: fünfter Filter
- 167: Filtermittelachse
- 171: Endoskopsystem
- 173: Endoskop
- 175: Endoskopaufnahme
- 177: Kamerakopf
- 179: Kameraaufnahme

## Patentansprüche

1. Filterwechselvorrichtung (101) für eine endoskopische Kamera, wobei die Filterwechselvorrichtung (101) ein Gehäuse (103) mit mindestens einem ersten Gehäuseteil (107, 109), einen optischen Durchgang (113) entlang einer optischen Achse (115), ein drehbares Antriebszahnrad (121) und mindestens zwei Filterhalterarme (131, 131, 133, 134, 135) mit jeweils einer Filteraufnahme (141, 142, 143, 144, 145) für einen optischen Filter (161, 162, 163, 164, 165) mit einer Filtermittelachse (167) aufweist, wobei die mindestens zwei Filterhalterarme (131, 131, 133, 134, 135) jeweils mittels einer Rotationswelle (137) mit einer Rotationsachse (139) drehbar an dem ersten Gehäuseteil (107, 109) angeordnet sind, wobei die jeweilige Rotationsachse (139) beabstandet zu der Filtermittelachse (167) des jeweiligen Filterhalterarms (131, 131, 133, 134, 135) ist und die Rotationsachsen (139) parallel zueinander angeordnet sind, **dadurch gekennzeichnet, dass** an jedem Filterhalterarm (131, 131, 133, 134, 135) ein Filterzahnrad (127) um die jeweilige Rotationswelle (137) angeordnet ist und das drehbare Antriebszahnrad (121) in das jeweilige Filterzahnrad (127) eingreifbar ist, sodass bei einem alleinigen Antreiben des drehbaren Antriebszahnrades (121) mittels Eingreifen in die Filterzahnräder (127) die mindestens zwei Filterhalterarme (131, 131, 133, 134, 135) gleichzeitig drehbar und/oder in den optischen Durchgang (113) einschwenkbar und/oder ausschwenkbar sind.

2. Filterwechselvorrichtung (101) nach Anspruch 1, **dadurch gekennzeichnet, dass** die Filterwechselvorrichtung (101) einen dritten Filterhalterarm, einen vierten Filterhalterarm, einen fünften Filterhalterarm und/oder optional weitere Filterhalterarme (131, 131, 133, 134, 135) aufweist.

3. Filterwechselvorrichtung (101) nach Anspruch 1 oder 2, **dadurch gekennzeichnet, dass** ein Abstand (152) zwischen der Filtermittelachse (167) und der Rotationsachse (139) eines Filterhalterarms (131, 131, 133, 134, 135) das 0,80-Fache bis 1,10-Fache eines maximalen Außendurchmessers (151) des Filterhalterarms (131, 131, 133, 134, 135) im Bereich der Filteraufnahme (141, 142, 143, 144, 145) ist.

4. Filterwechselvorrichtung (101) nach einem der vorherigen Ansprüche, **dadurch gekennzeichnet, dass** ein Abstand (153) zwischen zwei Rotationsachsen (139) zweier Filterhalterarme (131, 131, 133, 134, 135) das 1,05-Fache bis 1,50-Fache eines maximalen Außendurchmesser (151) des jeweiligen Filterhalterarms (131, 131, 133, 134, 135) im Bereich der Filteraufnahme (141, 142, 143, 144, 145) ist.

5. Filterwechselvorrichtung (101) nach einem der vorherigen Ansprüche, **dadurch gekennzeichnet, dass** der jeweilige Filterhalterarm (131, 131, 133, 134, 135) einen ersten Armabschnitt (147) mit der angeordneten Rotationswelle (137) und einen zweiten Armabschnitt (149) mit der angeordneten Filteraufnahme (141, 142, 143, 144, 145) aufweist, wobei beide Armabschnitte (147, 149) mittels eines Übergangsabschnittes (148) verbunden sind.

6. Filterwechselvorrichtung (101) nach Anspruch 5, **dadurch gekennzeichnet, dass** der erste Armabschnitt (147) und der zweite Armabschnitt (149) in einer Richtung entlang der Rotationsachse (139) zueinander beabstandet ausgebildet sind.

7. Filterwechselvorrichtung (101) nach Anspruch 5 oder 6, **dadurch gekennzeichnet, dass** der zweite Armabschnitt (149) in einer Richtung entlang der Rotationsachse (139) auf einer größeren Höhe als eine Höhe des ersten Armabschnittes (147) angeordnet ist, sodass ein zweiter Armabschnitt (149) eines ersten Filterhalterarms (131, 131, 133, 134, 135) zumindest teilweise über einen ersten Armabschnitt (147) eines zweiten Filterhalterarms (131, 131, 133, 134, 135) anordenbar und/oder drehbar ist.

8. Filterwechselvorrichtung (101) nach einem der Ansprüche 5 bis 7, **dadurch gekennzeichnet, dass** eine Differenz (155) zwischen einer maximalen Höhe des zweiten Armabschnittes (149) und einer maximalen Höhe des ersten Armabschnittes (147) ein 1,00-Faches bis 1,50-Faches einer maximalen Materialstärke (154) des zweiten Armabschnittes (149) in einer Richtung entlang der Rotationsachse (139) ist.

9. Filterwechselvorrichtung (101) nach einem der vorherigen Ansprüche, **dadurch gekennzeichnet, dass** die jeweilige Rotationwelle (137) mittels eines Sicherungselement (159) an dem mindestens einem Gehäuseteil (107) verbunden ist.

10. Filterwechselvorrichtung (101) nach einem der vorherigen Ansprüche, **dadurch gekennzeichnet, dass** das Antriebszahnrad (121) eine Innenverzahnung (123) und/oder eine Außenverzahnung (125) aufweist.

11. Filterwechselvorrichtung (101) nach einem der vorherigen Ansprüche, **dadurch gekennzeichnet, dass** die Filterwechselvorrichtung (101) ein zweites Gehäuseteil (109) aufweist und das erste Gehäuseteil (107) und das zweite Gehäuseteil (109) in ihrem Inneren oder an ihrem Äußeren miteinander verbunden sind, sodass das Antriebszahnrad (121) über seinen gesamten Umfang von außen frei antreibbar und/oder drehbar ist.

12. Filterwechselvorrichtung (101) nach einem der vorherigen Ansprüche, **dadurch gekennzeichnet, dass** die Filterwechselvorrichtung (101) einen Motor zum Antreiben des drehbaren Antriebszahnrades (121) aufweist.

13. Kamerakopf (177) für ein Endoskop (173), wobei der Kamerakopf (177) einen Bildsensor, eine Öffnung zum Empfangen von Licht eines Bildes entlang eines optischen Pfades und ein optisches Linsensystem zum Fokussieren des Lichtes auf den Bildsensor aufweist, **dadurch gekennzeichnet, dass** der Kamerakopf (177) zumindest eine Filterwechselvorrichtung (101) nach einem der Ansprüche 1 bis 12 aufweist.

14. Nachrüstsatz zum Nachrüsten eines Kamerakopfes und/oder eines Endoskopes, **dadurch gekennzeichnet, dass** der Nachrüstsatz zumindest eine Filterwechselvorrichtung (101) nach einem der Ansprüche 1 bis 12 aufweist, sodass die Filterwechselvorrichtung (101) zwischen einem proximalen Ende des Endoskopes (173) und einem distalen Ende des Kamerakopfes (177) anordenbar ist.
